**Europäisches Patentamt**

**European Patent Office**

**Office européen des brevets**

(19)

(11) Publication number : **0 335 970 B1**

## (12) EUROPEAN PATENT SPECIFICATION

(45) Date of publication of patent specification :
02.09.92 Bulletin 92/36

(51) Int. Cl.⁵ : **A61K 31/56, A61K 47/00**

(21) Application number : **89900392.5**

(22) Date of filing : **03.10.88**

(86) International application number :
**PCT/US88/03406**

(87) International publication number :
**WO 89/02742 06.04.89 Gazette 89/08**

(54) **TABLET FOR USE IN THE TREATMENT OF PROGESTERONE DEFICIENCY.**

(30) Priority : **05.10.87 US 104808**
**10.08.88 US 230616**

(43) Date of publication of application :
**11.10.89 Bulletin 89/41**

(45) Publication of the grant of the patent :
**02.09.92 Bulletin 92/36**

(84) Designated Contracting States :
**AT BE CH DE FR GB IT LI LU NL SE**

(56) References cited :
**EP-A- 0 168 044**
**AT-B- 385 654**
**DE-A- 2 659 251**
**GB-A- 1 595 185**
**US-A- 2 880 135**
**US-A- 2 895 881**

(56) References cited :
**US-A- 3 193 457**
**US-A- 3 402 240**
**US-A- 3 459 850**
**US-A- 3 535 419**
**US-A- 3 920 630**
**US-A- 4 159 346**
**US-A- 4 209 513**
**US-A- 4 439 432**

(73) Proprietor : **PHARMAGYN, INC.**
**3517 Hamlin Circle**
**Chamblee, GA 30341 (US)**

(72) Inventor : **ABRAHAM, Guy E.**
**5 Open Brand Road**
**Rolling Hills, CA 90274 (US)**

(74) Representative : **Hepworth, John Malcolm et al**
**J.M. Hepworth & Co. 36 Regent Place**
**Rugby Warwickshire CV21 2PN (GB)**

EP 0 335 970 B1

## Description

This invention relates generally to the administration of progesterone in the treatment of progesterone deficiency in the human female, and particularly to tabletized progesterone compositions.

Progesterone is a naturally occurring steroid which is biosynthesized in the ovaries and the adrenal cortices in nonpregnant women. Progesterone is medically administered in the treatment of progesterone deficiency as well as in the treatment of other disorders such as pregnancy complications and menstrual abnormalities. Even though it has been synthesized commercially since 1934, its clinical usefulness has been limited because of its extensive degradation by the liver following ingestion and because of its short shelf life.

Orally administered, synthetic progestational agents known as progestins, which do not degrade rapidly, have been used for treatment of some disorders. They however produce undesirable side effects. Thus efforts have continued to devise a manner to administer natural progesterone. Since intramuscular injection of progesterone is not therapeutically practical, and since vaginal and rectal administration is inconvenient and aesthetically displeasing, attempts have continued to develop a natural progesterone composition that can be administered orally.

Heretofore, two general approaches have been taken in attempts to circumvent the effect that the liver has on orally administered progesterone. One involves bypassing the liver by giving oily preparations of progesterone to encourage its absorption through the lymphatic system. Laboratoires Besins Iscovesco of Paris, France has followed this approach by developing a soft gelatin capsule marketed in Europe under the name Utrogestan. It has progesterone combined with a vegetable oil in gelatin. Its effectiveness however has been limited since serum levels of progesterone following its administration have been found to be erratic, non predictable and not characterized by sustained release. Moreover, its production is messy and inefficient by being encapsulated in gelatin. The Besins progesterone-containing capsules are disclosed in DE-A-2 659 251 and the corresponding GB-1 595 185B.

The other approach is to micronize the progesterone by placing it in powdered form in an environment that creates breakage of the particles to very small sizes, mostly under 10 µm (microns). In micronized form it is absorbed so rapidly that massive dosages saturate the metabolic capacity of the liver to a point that a significant amount can go through the liver without breakdown. Massive dosages for any significant period, however, would be both clinically harmful and not economically feasible.

Accordingly, it is seen that a need remains for a pharmaceutically product by which natural progesterone may be orally administered with effective absorption rates, improved shelf life, with sustained release properties and in moderate dosages.

From US-A-3 402 240 pharmaceutical tablets comprising a tabletized mixture of an active ingredient in powder form and a wax in powdered form having a melting point above body temperature are known.

It has now been discovered that a tabletized mixture of micronized progesterone and a wax is effective in elevating serum levels of progesterone. The concentration of the wax is preferably between 20% and 150% by weight of that of the progesterone. The particle size of the progesterone is largely below lOµm(microns). By thoroughly blending micronized natural progesterone with a wax having a melting point above body temperature, such as carnauba wax, degradation of the progesterone by the liver is sufficiently limited so as to achieve good serum level increases in progesterone on a sustained and substantially predictable basis. The inclusion of a limited quantity of safflower oil has also been found to be beneficial in this regard.

EXAMPLE I

| | |
|---|---|
| micronized progesterone | 1000 grams |
| Brazilian carnauba wax | 1000 grams |
| Avicel (registered Trade Mark) 102 | 1000 grams |
| microsilica gel | 15 grams |
| stearic acid | 30 grams |
| AC DI SOL (registered Trade Mark) | 30 grams |
| magnesium stearate | 15 grams |

The preparation is made by placing the progesterone in micronized, powdered form having particle sizes less than about 10 µm (microns) into a blender with the powdered Brazilian carnauba wax and blending the mixture for 15 minutes. The Avicel 102, AC DI SOL, the microsilica gel and stearic acid are premixed to a uniform blend and then thoroughly blended with the progesterone and wax mixture for 10 minutes. Following this the magnesium stearate is added to the mixture and blended for another 5 minutes. The blend is then conventionally compressed to form tablets that are stored under refrigeration at about 4.4°C (40°F) or less.

Clinical studies have found that the oral ingestion during the midluteal phase of the menstrual cycle of tablets having 100 milligrams of progesterone in the morning and having 200 milligrams of progesterone at bedtime, of the Example I composition, increases the serum concentration of progesterone for sustained periods of time sufficient to evoke progestinal responses in the responsive end organs. Clinical tests have shown the following serum levels to be achieved:

## TABLE I

### Dosage in milligrams

|  | 100 | 200 |
|---|---|---|
| Peak Time (hours) | 4-5 | 4-5 |
| Peak concentration (ng/ml) | 3.9 | 10.2 |
| Range at peak (ng/ml) | 1.8-5.9 | 4.7-18.5 |
| Surface area under curve* | 43 | 100 |
| No. participants | 9 | 5 |

*Curve being a plot of serum progesterone in ng/ml against time in hours.

Though the physiological mechanism at work here is still not fully understood, apparently the presence of the wax finely blended with progesterone in micronized form limits the effectiveness of the liver in degrading the progesterone during liver transit. Thus, micronized progesterone enters into the bowel in sufficient quantity to be available for absorption at effective rates with the administration of only the three tablets per day of 100 mg progesterone each. Not only does it enter the bowel in sufficient quantity but it also is present there for release from the wax at good sustained release rates.

The Avicel 102 cellulose filler is added to provide bulk. It should be present in a concentration of 20% to 50% of total weight. The AC DI SOL, a specialized cellulose filler, is provided at 1/2% to 1% of total weight as a disintegrant. The stearic acid and magnesium stearate serve as lubricants to prevent adherence of the composition to the tabletizing apparatus. The stearic acid and magnesium stearate are each provided at 1% to 2% of total tablet weight. The microsilica gel acts as a desiccant and flowing agent and should preferably be present from 1% to 2% of total weight. The wax must have a melting point above body temperature and should be at a concentration of 20% to 150% by weight of the progesterone. Concentrations of less than 20% suffer from adverse losses of absorption and of sustain release properties. Concentrations above 150% restrict absorption of the progesterone from the intestinal track.

EXAMPLE II

| | |
|---|---|
| micronized progesterone | 200 mg |
| Brazilian carnauba wax | 100 mg |
| silica-based excipient* | 400 mg |
| safflower oil | 50 mg |
| silica powder | 2% |
| stearic acid | 1% |
| magnesium stearate | 1% |

*Micosolle, available from Biomicotek, Inc. of Torrance, California

Post-menopausal women were orally administered 200 mg of the preparation in tablet form while fasting. Blood samples were obtained hourly for 6 hours, then at 8 hours and 24 hours. Serum progesterone was measured by radioimmunoassay and bioavailability was assessed by measuring the area under the curve of serum progesterone levels for 8 hours. For comparison, post-menopausal women were orally administered the same preparation with zero safflower oil content and with 200 mg safflower oil. The results are shown in Table II.

## TABLE II

| Safflower oil/ progesterone ratio | Mean surface area under curve to 8 hours* | Number of Subjects |
|---|---|---|
| 0 | 100 | 5 |
| 0.25 | 249 | 12 |
| 1.0 | 187 | 8 |

*Curve being a plot of serum progesterone in ng/ml against time in hours.

It thus is seen that the bioavailability of orally ingested micronized progesterone was increased significantly with the addition of safflower oil in limited quantities. Preferably, in the compositions of the invention safflower oil is present at a concentration not exceeding that of the progesterone concentration.

The preparations with safflower oil are prepared by mixing micronized progesterone with the safflower oil and then adding the carnauba wax. The excipient Micosolle is added to provide sufficient hardness to the tablet and for flowability of the powder during compression. The magnesium stearate, stearic acid and silica are then added, thoroughly mixed and the preparation conventionally compressed.

## Claims

1. A pharmaceutical composition suitable for orally administering progesterone comprising a tabletized mixture of micronized progesterone in powdered form and a wax in powdered form having a melting point above body temperature.

2. The composition of claim 1 wherein said progesterone has particle sizes generally less than 10 $\mu$m.

3. The composition of claim 1 wherein said wax is present at a concentration of between 20% and 150% by weight that of said progesterone.

4.   The composition of claim 1 wherein said wax is carnauba wax

5.   The composition of claim 1 further comprising a cellulose filler in an amount of between 20% and 50% of total composition weight.

6.   The composition of claim 1 further comprising 1% to 2% of total weight of magnesium stearate as a lubricating agent.

7.   The composition of claim 1 further comprising 1% to 2% of total weight of microsilica gel as a flow agent.

8.   The composition of claim 1 further comprising 1% to 2% of total composition weight of stearic acid as a lubricant.

9.   The composition of claim 1 further comprising safflower oil.

10.   The composition of claim 9 wherein safflower oil is present at a concentration not exceeding that of the progesterone concentration.

11.   A method of making a tablet for oral ingestion to elevate blood level contents of progesterone wherein the method comprises the steps of (a) mixing micronized progesterone in powder form with a wax in powdered form; (b) blending the mixture of progesterone and wax; (c) adding a filler in powdered form to the mixture; (d) blending the mixture of progesterone, wax and filler, and (3) compressing the blended mixture.

12.   The method of claim 11 wherein step (a) progesterone in powder form is mixed with carnauba wax in powder form.

13.   The method of claim 11 wherein step (a) progesterone in powder form of a particle size less than 10 $\mu$m (microns) is mixed with wax in powder form.

14.   The method of claim 11 wherein step (a) the powdered wax is mixed with powder progesterone in a concentration of between 20% and 150% by weight of the progesterone.

15.   The method of claim 11 wherein step (c) a cellulose filler is added to the mixture in an amount between 20% and 50% by weight of the total weight of the composition.

16.   The use of a tablet comprised of a mixture of powdered micronized progesterone and powdered wax with the concentration of max in the tablet being sufficient to achieve rendered sustained release of progesterone after passage through the liver for the manufacture of a medicament for treating progesterone deficiency in the human female by oral administration.

17.   The use of claim 16 for oral administration during the midluteal phase of the menstrual cycle.

18.   The use of claim 16 wherein the tablet also comprises safflower oil.

19.   The method of making a tablet for oral ingestion to elevate blood level contents of progesterone wherein the method comprises the steps of:
     (a) mixing micronized progesterone in powder form with safflower oil;
     (b) adding carnauba wax and blending the mixture of progesterone, safflower oil and wax;
     (c) adding an excipient; and
     (d) blending and compressing the blended mixture in tablet form.

## Revendications

1.   Composition pharmaceutique appropriée pour administrer de la progestérone par voie orale, comprenant un mélange en forme de comprimé de progestérone micronisée sous forme pulvérulente et d'une cire sous forme pulvérulente ayant un point de fusion au-dessus de la température du corps.

2.   Composition selon la revendication 1, dans laquelle ladite progestérone possède des granulométries généralement inférieures à 10 $\mu$m.

3. Composition selon la revendication 1, dans laquelle ladite cire est présente en une concentration entre 20% et 150% en poids de ladite progestérone.

4. Composition selon la revendication 1, dans laquelle ladite cire est la cire de carnauba.

5. Composition selon la revendication 1, comprenant, en outre, une matière de charge de cellulose en une quantité entre 20% et 50% du poids total de la composition.

6. Composition selon la revendication 1, comprenant, en outre, du stéarate de magnésium à raison de 1% à 2% du poids-total, comme agent lubrifiant.

7. Composition selon la revendication 1, comprenant, en outre, du gel de microsilice à raison de 1% à 2% du poids total, comme agent d'écoulement.

8. Composition selon la revendication 1, comprenant, en outre, de l'acide stéarique à raison de 1% à 2% du poids total de la composition, comme lubrifiant.

9. Composition selon la revendication 1, comprenant, en outre, de l'huile de carthame.

10. Composition selon la revendication 9, dans laquelle l'huile de carthame est présente en une concentration n'excédant pas la concentration de progestérone.

11. Procédé de préparation d'un comprimé pour une ingestion par voie orale dans le but d'élever le taux de concentration de progestérone dans le sang, dans lequel le procédé comprend les étapes consistant à : (a) mélanger de la progestérone micronisée sous forme pulvérulente à une cire sous forme pulvérulente; (b) homogénéiser le mélange de progestérone et de cire; (c) ajouter une matière de charge sous forme pulvérulente au mélange; (d) homogénéiser le mélange de progestérone, de cire et de matière de charge; et (e) comprimer le mélange homogénéisé.

12. Procédé selon la revendication 11, dans lequel, à l'étape (a), on mélange la progestérone sous forme pulvérulente avec de la cire de carnauba sous forme pulvérulente.

13. Procédé selon la revendication 11, dans lequel, à l'étape (a), on mélange de la progestérone sous forme pulvérulente ayant une granulométrie inférieure à 10 µm (microns), à de la cire sous forme pulvérulente.

14. Procédé selon la revendication 11, dans lequel, à l'étape (a), on mélange la cire pulvérulente avec de la progestérone pulvérulente en une concentration entre 20% et 150% en poids de la progestérone.

15. Procédé selon la revendication 11, dans lequel, à l'étape (c), on ajoute au mélange une matière de charge de cellulose en une quantité entre 20% et 50% en poids du poids total de la composition.

16. Utilisation d'un comprimé comprenant un mélange de progestérone micronisée pulvérulente et de cire pulvérulente, la concentration de cire dans le comprimé étant suffisante pour obtenir une libération prolongée de progestérone après son passage à travers le foie, pour la préparation d'un médicament destiné à traiter la carence en progestérone chez les êtres humains de sexe féminin par administration par voie orale.

17. Utilisation selon la revendication 16, pour une administration par voie orale au cours de la phase lutéale médiane du cycle menstruel.

18. Utilisation selon la revendication 16, dans laquelle le comprimé comprend également de l'huile de carthame.

19. Procédé de préparation d'un comprimé pour une ingestion par voie orale dans le but d'élever les taux de concentration de progestérone dans le sang, dans lequel le procédé comprend les étapes consistant à :
   (a) mélanger de la progestérone micronisée sous forme pulvérulente à de l'huile de carthame;
   (b) ajouter de la cire de carnauba et homogénéiser le mélange de progestérone, d'huile de carthame et de cire;
   (c) ajouter un excipient; et
   (d) homogénéiser et comprimer le mélange homogénéisé sous forme de comprimés.

**Patentansprüche**

1. Eine pharmazeutische Zusammensetzung, geeignet um Progesteron oral zu verabreichen, enthaltend eine Mischung in Tablettenform von miniaturisiertem Progesteron in Pulverform und einem Wachs in Pulverform, das einen Schmelzpunkt über der Körpertemperatur hat.

2. Zusammensetzung nach Anspruch 1, wobei das Progesteron Partikelgrößen im allgemeinen kleiner als 10 μm hat.

3. Zusammensetzung nach Anspruch 1, wobei das Wachs in Konzentrationen zwischen 20 und 150 Gewichtsprozent des Progesterons vorliegt.

4. Zusammensetzung nach Anspruch 1, wobei das Wachs Carnauba Wachs ist.

5. Zusammensetzung nach Anspruch 1, die zusätzlich Cellulose Füllmaterial in einer Menge zwischen 20 und 50% des Gesamtgewichts enthält.

6. Zusammensetzung nach Anspruch 1, die zusätzlich Magnesiumstearat in einer Menge von 1 bis 2% des Gesamtgewichts als Gleitmittel enthält.

7. Zusammensetzung nach Anspruch 1, die zusätzlich Microsilica Gel in einer Menge von 1 bis 2% des Gesamtgewichts als Flußmittel enthält.

8. Zusammensetzung nach Anspruch 1, die zusätzlich Stearinsäure in einer Menge von 1 bis 2% des Gesamtgewichts als Gleitmittel enthält.

9. Zusammensetzung nach Anspruch 1, die zusätzlich Karthamusöl enthält.

10. Zusammensetzung nach Anpruch 9, wobei das Karthamusöl in einer Konzentration, die die Progesteronkonzentration nicht übersteigt, vorliegt.

11. Verfahren zur Herstellung einer Tablette für die orale Aufnahme, um den Blutspiegel von Progesteron zu erhöhen, wobei das Verfahren folgende Schritte enthält: (a) Mischen des miniaturisierten Progesterons in pulverisierter Form mit einem Wachs in pulverisierter Form; (b) gründliches Vermengen der Progesteron-Wachs Mischung; (c) Zugeben eines Füllstoffes in pulverisierter Form zu der Mischung; (d) Vermengen der Progesteron -Wachs-Füllstoff Mischung; und (e) Verdichten der vermengten Mischung.

12. Verfahren nach Anspruch 11, wobei im Schritt (a) das Progesteron in pulverisierter Form mit Carnauba Wachs in pulverisierter Form gemischt wird.

13. Verfahren nach Anspruch 11, wobei im Schritt (a) das Progesteron in pulverisierter Form eine Partikelgröße kleiner als 10 μm hat und mit Wachs in pulverisierter Form gemischt wird.

14. Verfahren nach Anspruch 11, wobei im Schritt (a) das pulverisierte Wachs mit pulverisiertem Progesteron in einer Konzentration von 20 bis 150 Gewichtsprozent des Progesterons gemischt wird.

15. Verfahren nach Anspruch 11, wobei im Schritt (c) ein Cellulose-Füllmaterial in einer Menge von 20 bis 50 Gewichtsprozent des Gesamtgewichts der Zusammensetzung zur Mischung hinzugefügt wird.

16. Verwendung einer Tablette, die eine Mischung von pulverisiertem, miniaturisiertem Progesteron und pulverisiertem Wachs enthält, mit einer Konzentration des Wachses in der Tablette, die genügt, um ausreichende Langzeitwirkung des Progesterons nach Passage durch die Leber zu gewährleisten, zur Herstellung eines Medikaments für die Behandlung von Progesteronmangel bei Frauen durch orale Verabreichung.

17. Verwendung nach Anspruch 16 für die orale Verabreichung während der Mitte der lutealen Phase im Menstruationszyklus.

18. Verwendung nach Anspruch 16, wobei die Tablette außerdem Karthamusöl enthält.

19. Verfahren zur Herstellung einer Tablette für die orale Aufname, um den Blutspiegel von Progesteron zu erhöhen, wobei das Verfahren die folgenden Schritten enthält: (a) Mischen des miniaturisierten Progesterons in pulverisierter Form mit Karthamusöl; (b) Zugeben von Carnauba Wachs und Vermischen von Progesteron, Karthamusöl und Wachs; (c) Zugeben eines Trägers; und (d) gründliches Vennengen und Verdichten der Mischung in Tablettenform.